(19) Europäisches Patentamt / European Patent Office / Office européen des brevets

(11) **EP 3 763 398 A1**

(12) **EUROPEAN PATENT APPLICATION**
published in accordance with Art. 153(4) EPC

(43) Date of publication:
**13.01.2021 Bulletin 2021/02**

(51) Int Cl.:
*A61L 27/60* (2006.01)  *A61L 27/40* (2006.01)
*A61L 27/14* (2006.01)  *C08L 101/00* (2006.01)
*C08J 3/05* (2006.01)  *C08G 81/00* (2006.01)

(21) Application number: **18920157.7**

(22) Date of filing: **25.05.2018**

(86) International application number:
**PCT/KR2018/005988**

(87) International publication number:
**WO 2019/225789 (28.11.2019 Gazette 2019/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.05.2018 KR 20180058947**

(71) Applicant: **Dexlevo Inc.**
**Seoul 08504 (KR)**

(72) Inventors:
• **YU, Jae Won**
  **Incheon 22675 (KR)**
• **SHIM, Myung Seob**
  **Seoul 06673 (KR)**
• **KIM, Jun Bae**
  **Daejeon 34049 (KR)**

(74) Representative: **Swindell & Pearson Limited**
**48 Friar Gate**
**Derby DE1 1GY (GB)**

(54) **COMPOSITION FOR TISSUE REPAIR AND MANUFACTURING METHOD THEREFOR**

(57) Disclosed in the present invention are: a composition for tissue repair using a nontoxic biocompatible polymer; and a manufacturing method therefor. Provided according to the present invention are: a composition for tissue repair, the composition comprising a copolymer obtained from the polymerization of a hydrophobic biocompatible polymer and a hydrophilic biocompatible polymer, the composition being in a colloidal phase in which the copolymer is dispersed in water; and a manufacturing method therefor.

FIG. 2

EP 3 763 398 A1

**Description**

<u>**BACKGROUND**</u>

The present disclosure relates to a composition for tissue repair treatment and a method for manufacturing the same, and more particularly, to a composition for tissue repair treatment and a method for manufacturing the same using polymers.

As a social structure is changing and the population is increasing, the number of patients with burns, a decubitus, trauma, plastic surgery, intractable ulcer, diabetic dermonecrosis, *etc.* is gradually increasing. Thus, a method for treating damaged skins is being developed accordingly. Even 30 years ago, patients whose damaged skin occupies 60% or more of a body surface area by burns usually died of sepsis, but recently improved artificial skin can protect dehydration and infections, and therefore, mortality rate can be significantly decreased. The artificial skin is largely divided into wound dressing and cultured skin.

The wound dressing is applicable to topical wounds or wounds depth less severe, and the wound dressing plays a role in protecting a wound during a period of time until skin grafting is possible or during 3-4 weeks until auto cultured skin is completed, and thereby easily applying cultured skin.

The cultured skin is used in treatment for minimizing scar tissue in the case of severe skin loss or extensive wound. The cultured skin is grafted for permanent engraftment after fully proliferating dermal cells using cell culture techniques. For cultured skin, many reviews for safety are required through a test of bacteria, fungi, endotoxicity, and mycoplasma, and it is inconvenient in that the cultured skin should be manufactured after confirming safety through various viruses [HIV 1&2, HTLV I I & II II, CMV lgM, Hepatitis B & C, and adenovirus] test. Further, in the case of grafting skin from human corpses, there are a problem of unknown source thereof and a disadvantage that, it is impossible to thermally treat the component in the human body in a processing treatment, the fatal viruses aforementioned cannot be sterilized up to 100%. Furthermore, since it takes at least one week to culture cells and graft the same, it is hard to be used for patients requiring first aid.

Meanwhile, the wound dressing has an advantage that the wound dressing can be extensively applied and easily treated relative to the cultured skin, and can be applied to patients requiring first aid. The wound dressing, however, is hard to be grafted permanently since the wound dressing is used for temporary covering. In addition, the wound dressing of natural polymer, such as chitin, chitosan, collagen has a low mechanical strength, is expensive, and is hard to mass produce; and the wound dressing of synthetic polymer, such as silicone and polyurethane has a disadvantage that the wound dressing has low affinity with cells and no adhesion to a wound site.

Recently, several products using hyaluronic acid gel have been developed, but hyaluronic acid is resorbed very rapidly in the living body between 2 weeks and 2 months, thereby causing a problem. Thus, a product the resorbing period of which is extended by crosslinking hyaluronic acid and crosslinkable materials with each other, as disclosed in Korean Patent Laid-open Publication No. 10-2014-0072008, is commercially available. Such a crosslinked product, however, is also reported to have a problem due to toxicity of crosslinkable materials.

Due to these problems, recently several products for tissue repair treatment using biodegradable polymers are developed, and these products are developed and used as a filler formulation using the existing biocompatible polymers, or a formulation which is dispersed through media having viscosity after processing polymers insoluble in water into microparticles. A formulation in which 20-50 μm of poly lactic acid (PLA) particles are dispersed in carboxymethylcellulose (CMC) aqueous solution, or a formulation in which 20-50 μm of polycaprolactone (PCL) particles are dispersed in CMC and glycerin aqueous solution has been used; however, this causes an inconvenience on a procedure because microparticles to be blocked by a needle during injection, and also arises a problem in that microparticles are not uniformly dispersed, so that tissues are not uniformly repaired.

Further, according to Klaus Laeschke, 'Biocompatibility of Microparticles into Soft Tissue Fillers', "Semin Cutan Med Surg 23", 2004, 214-217, a polymer-based tissue repair treatment product should have a particle diameter of 40 μm or more to exhibit long-lasting effects, while avoiding phagocytosis in vivo. However, using the formulation having a particle diameter of 40 μm or more results in an inconvenience on a procedure because microparticles are blocked by a needle, and causes a problem in that microparticles are not uniformly dispersed, so that tissues are not uniformly repaired.

Development of a product for tissue repair treatment to solve such problems above is needed urgently.

<u>**SUMMARY**</u>

The present disclosure has been made keeping in mind the above problems occurring in the related art, and directed to providing a composition for tissue repair treatment using non-toxic polymers and a method for manufacturing the same.

The present disclosure provides a composition for tissue repair treatment, including a copolymer in which a hydrophobic biocompatible polymer and a hydrophilic biocompatible polymer are polymerized, the composition having a colloidal phase in which the copolymer is dispersed in water.

Further, the present disclosure provides a composition for tissue repair treatment, wherein the composition may have a range of K factor represented by the following equation 1 is 0.3-1.8:

<Equation 1>

$$K = (m_{100} * M_h^2 * 10)/(M_l * HLB^2)$$

In equation 1, $m_{100}$ is the number of moles of polymers in 100 g of an aqueous solution, $M_h$ is the molecular weight of a hydrophilic part, $M_l$ is the molecular weight of a hydrophobic part, and HLB is represented by the following equation 2,

<Equation 2>

$$HLB = 20 * M_h/M$$

In equation 2, $M_h$ is the molecular weight of the hydrophilic part, and M is the total molecular weight.

In addition, the present disclosure provides a composition for tissue repair treatment, wherein the value of HLB may be 1-14 in the equation 2.

Furthermore, the present disclosure provides a composition for tissue repair treatment, wherein the hydrophobic biocompatible polymer may be at least any one polymer selected from the group consisting of polyglycolic acid, polycaprolactone, poly lactic acid, polydioxanone, poly(trimethylene carbonate), polyhydroxybutyrate, and a copolymer including the same.

Additionally, the present disclosure provides a composition for tissue repair treatment, wherein the hydrophilic biocompatible polymer comprises a glycol compound.

In addition, the present disclosure provides a composition for tissue repair treatment, wherein the glycol compound may be at least any one polymer selected from the group consisting of methoxy polyethylene glycol, dihydroxy polyethylene glycol, mono-alkoxy polyethylene glycol, and polyethylene glycol.

Moreover, the present disclosure provides a composition for tissue repair treatment, wherein the bonding structure of the copolymer may include the structure of the following formula 1, formula 2, or formula 3:

[Formula 1]

X-Y

[Formula 2]

Y-X-Y

[Formula 3]

X-Y-X

In formulae 1-3, X is the hydrophilic biocompatible polymer, and Y is the hydrophobic biocompatible polymer.

Further, the present disclosure provides a composition for tissue repair treatment, wherein the hydrophilic biocompatible polymer may be molecular 300-20,000 g/mol.

In addition, the present disclosure provides a composition for tissue repair treatment, wherein the hydrophobic biocompatible polymer may be molecular 1,000-30,000 g/mol.

Furthermore, the present disclosure provides a composition for tissue repair treatment, wherein the copolymer may be molecular 1,300-50,000 g/mol.

Moreover, the present disclosure provides a composition for tissue repair treatment, wherein the concentration of the copolymer in a colloidal solution may be 10-50 wt%.

Additionally, the present disclosure provides a composition for tissue repair treatment, wherein the colloidal solution may have no change or an increase in turbidity when water is added.

Further, the present disclosure provides a method for manufacturing a composition for tissue repair treatment, the method including: preparing a polymer by polymerizing a hydrophobic biocompatible polymer and a hydrophilic biocompatible polymer; and obtaining a colloidal solution by adding the polymer to water.

The present disclosure may provide a colloidal phase composition for tissue repair treatment including a copolymer in which a hydrophobic biocompatible polymer and a hydrophilic biocompatible polymer are polymerized, non-toxic and safe when the composition is injected in the living body, capable of applying to emergency patients.

**BRIEF DESCRIPTION OF THE DRAWINGS**

Fig. 1 is a picture which is taken with DSLR (D3000, Nikon, Japan) to confirm whether samples are leaked after PBS and a colloidal aqueous solution are injected;

Fig. 2 is a picture, which is taken through an optical microscope, and shows a skin thickness over time after a colloidal aqueous solution is injected;

Fig. 3 is a picture, which is taken through an optical microscope, and shows collagen over time after a colloidal aqueous solution is injected;

Fig. 4 is a picture, which is taken through an optical microscope, and shows a skin thickness over time after PBS is injected;

Fig. 5 is a picture, which is taken through an optical microscope, and shows a skin thickness over time after PBS is injected;

Fig. 6 is a graph showing a skin thickness over time after PBS and a colloidal aqueous solution are injected; and

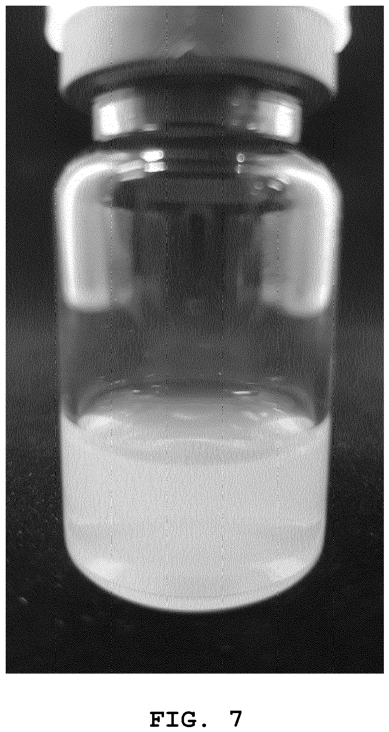

Fig. 7 is a picture, which is taken with DSLR (D3000, Nikon, Japan), and shows a colloidal aqueous solution.

**DETAILED DESCRIPTION OF EMBODIMENTS**

Hereinafter, the present disclosure will be described in detail with reference to the exemplary embodiments. All terms or words used in the specification and claims should not be construed as a general or dictionary definition but are to be construed meaning and concepts meeting the technical spirits of the present disclosure based on a principle that the inventors can appropriately define the concepts of terms in order to describe their own disclosures in best mode. Therefore, configurations described in embodiments of the present specification indicate only the most preferred example rather than indicating all the technical spirits of the present disclosure, and thus, it is to be understood that various equivalents and modifications that can replace the above configurations may be present. Further, throughout the specification, unless explicitly described to the contrary, the word "comprise", "include", "comprising", and/or "including" will be understood to imply the inclusion of stated elements but not the exclusion of any other elements.

The present inventors studied a biocompatible polymer to make a non-toxic, safe composition for tissue repair treatment which is capable of applying to emergency patients and being manufactured relatively inexpensively. As a result, it was observed that a copolymer in which a hydrophobic biocompatible polymer and a hydrophilic biocompatible polymer are polymerized can repair tissue safely without toxicity in vivo and apply to emergency patients, and the present disclosure was achieved.

Therefore, the present disclosure discloses a composition for tissue repair treatment, including a copolymer in which a hydrophobic biocompatible polymer and a hydrophilic biocompatible polymer are polymerized, and having a colloidal phase in which the copolymer is dispersed in water.

The term "colloidal phase" refers to a state in which fine particles larger than molecules or ions are dispersed in gas or liquid, and the term "colloid" refers to the whole that is in a colloidal phase.

Particle size of the existing filler products can be identified with the naked eye, but the particle size of the colloidal phase according to the present disclosure cannot be identified with the naked eye, and insoluble foreign substance does not present in the colloid. The insoluble foreign substance refers to insoluble foreign substance which is easily detected when a solution formulation is added to a container which is cleaned according to Insoluble Particulate Matter Test, General Tests of United States Pharmacopeia (USP), and is then observed with the naked eye in the position of a brightness of about 2750-3000 lx directly below a white light source.

In the present disclosure, the particle size cannot be identified with the naked eye, and when the composition is injected into the body, polymers are bonded to each other to form a matrix structure, thereby exhibiting a long-lasting effect of tissue repair treatment in the skin without phagocytosis.

The range of K factor represented by the following equation 1 of the composition according to the present disclosure may be 0.3-1.8, preferably 0.4-1.5. If the K factor is less than 0.3 or larger than 1.8, the efficacy as a formulation

may be decreased.

<Equation 1>

$$K=(m_{100}*M_h^2*10)/(M_l*HLB^2)$$

In equation 1, $m_{100}$ is the number of moles of polymers in 100 g of an aqueous solution, $M_h$ is the molecular weight of a hydrophilic part, $M_l$ is the molecular weight of a hydrophobic part, and HLB is represented by the following equation 2,

<Equation 2>

$$HLB=20*M_h/M$$

In equation 2, $M_h$ is the molecular weight of the hydrophilic part, and M is the total molecular weight.

In a colloidal aqueous solution in which a copolymer in which a hydrophobic biocompatible polymer and a hydrophilic biocompatible polymer are polymerized is dissolved in water, the number of moles of the copolymer dissolved in 100 g of the aqueous solution has a value varying with the molecular weight of the hydrophilic biocompatible polymer, the molecular weight of the hydrophobic biocompatible polymer, and the mixture ratio, and thus the range of the tissue repair treatment effect of the composition for tissue repair treatment according to the present disclosure could not be set. In the present disclosure, to derive the range of the tissue repair treatment effect of the composition for tissue repair treatment, in a colloidal aqueous solution in which the copolymer in which a hydrophobic biocompatible polymer and a hydrophilic biocompatible polymer are polymerized is dissolved in water, the correlation among the number of moles of the copolymer dissolved in 100 g of the aqueous solution, the hydrophilic biocompatible polymer, the hydrophobic biocompatible polymer, and HLB is studied. As a result, a constant value is identified and is defined as K factor.

In other words, the K factor in the present disclosure represents the correlation among the number of moles of the copolymer dissolved in 100 g of the aqueous solution, the molecular weight of the hydrophilic biocompatible polymer, the molecular weight of the hydrophobic biocompatible polymer, and HLB value in a colloidal phase in which a copolymer in which a hydrophobic biocompatible polymer and a hydrophilic biocompatible polymer are polymerized is dispersed.

The K factor in the colloidal phase represents a constant value according to the number of moles of the copolymer dissolved in 100 g of the aqueous solution, the molecular weight of the hydrophilic biocompatible polymer, the molecular weight of the hydrophobic biocompatible polymer, and the HLB.

The efficacy of a formulation means that, before injection into the body, a hydrophilic polymer in a copolymer plays a major role, without insoluble foreign substance which can be identified with the naked eye due to the interaction of a solvent and a polymer, to form a colloidal phase in which polymers are uniformly and stably dispersed in an aqueous solution, but, after injection into the body, a hydrophobic polymer plays a major role, due to the influence of the environment in the body, to collapse the structure in which the polymers are stably dispersed in the aqueous solution, and then a matrix structure formed by bonding polymers to each other induces collagen, thereby repairing tissue.

Further, the repairing tissue refers to a mechanism that, when necrosis and loss occur in a tissue due to trauma or inflammation of a skin tissue, etc., restores the tissue to the original state.

The molecular weight of a polymer in the present disclosure refers to number average molecular weight (Mn). The number average molecular weight means an average molecular weight obtained by averaging the molecular weight of the component molecules of a polymer compound having a molecular weight distribution by number fraction or mole fraction.

HLB value calculated by the equation 2 may be in a range of 1-14, preferably 2-12, and more preferably 2.5-10. If HLB value is less than 1, the polymerized copolymer may not be dissolved in water; and if HLB value is greater than 14, the composition is absorbed in the body during the injection of the composition into the body, so that the effect as a formulation cannot be exhibited.

The term "Hydrophile-Lipophile Balance (HLB) value" refers to an affinity for water and oil of an amphiphilic polymer. Large HLB indicates a high proportion of a hydrophilic polymer, and small HLB indicates a low proportion of a hydrophilic polymer.

In order to satisfy the K factor according to the equation 1, the hydrophobic biocompatible polymer may be at least any one polymer selected from the group consisting of polyglycolic acid, polycaprolactone, poly lactic acid, polydioxanone, poly(trimethylene carbonate), polyhydroxybutyrate, and a copolymer including the same, and preferably the hydrophobic biocompatible polymer may be polycaprolactone.

In order to satisfy the K factor according to the equation 1, the hydrophilic biocompatible polymer may comprise a glycol compound.

In order to satisfy the K factor according to the equation 1, the glycol compound may be at least any one polymer selected from the group consisting of methoxy polyethylene glycol, dihydroxy polyethylene glycol, mono-alkoxy polyethylene glycol, and polyethylene glycol, preferably methoxy polyethylene glycol.

The bonding structure of the copolymer may be, but is not limited to, represented by the structure of the following formula 1, formula 2, or formula 3:

[Formula 1]

$$X-Y$$

[Formula 2]

$$Y-X-Y$$

[Formula 3]

$$X-Y-X$$

In formulae 1-3, X is a hydrophilic biocompatible polymer, and Y is a hydrophobic biocompatible polymer.

The molecular weight of the hydrophilic biocompatible polymer in order to satisfy the K factor according to the equation 1 may be 300-20,000 g/mol, preferably 700-15,000 g/mol, and more preferably 1,000-10,000 g/mol.

The molecular weight of the hydrophobic biocompatible polymer in order to satisfy the K factor according to the equation 1 may be 1,000-30,000 g/mol, preferably 1,500-27,500 g/mol, and more preferably 2,000-25,000 g/mol.

In order to satisfy the K factor according to the equation 1, the molecular weight of the copolymer may be 1,300-50,000 g/mol, preferably 2,200-42,500 g/mol, and more preferably 3,000-35,000 g/mol.

In order to satisfy the K factor according to the equation 1, the concentration of the copolymer in the colloidal solution may be 10-50 wt%, preferably 13-48 wt%, and more preferably 15-45 wt%. If the concentration is more than 50 wt%, the colloidal aqueous solution becomes a gel phase having a very high viscosity, so it is very hard to be injected through a syringe, and if the concentration is less than 10 wt%, the effect as a formulation cannot be exhibited.

The colloidal phase has no change or an increase in turbidity when water is added. A general colloidal phase has a decrease in turbidity when water is added, but the turbidity of the colloidal phase in the present disclosure does not decrease. The polymer dispersed in the colloidal phase in the present disclosure forms a structure in which a hydrophilic biopolymer and a hydrophobic biopolymer can be dissolved together in water. When water is added, however, a soluble structure formed by a hydrophilic biopolymer and a hydrophobic biopolymer is collapsed. Therefore, when water is added as above, bonding between hydrophobic biopolymers is formed, so that the turbidity of the colloidal phase does not change or rather increase.

Another aspect of the present disclosure provides a method for manufacturing the composition for tissue repair treatment.

The method includes preparing a copolymer by polymerizing a hydrophobic biocompatible polymer and a hydrophilic biocompatible polymer, and obtaining a colloidal solution by adding the copolymer to water.

In particular, a colloidal solution in which the copolymer is dispersed in water, the particle size cannot be identified with the naked eye by heating to a temperature between the melting point of the copolymer and the boiling point of water, and a colloidal phase in which insoluble foreign substance does not present is formed.

When the composition for tissue repair treatment manufactured by the method is injected into the skin, the composition exhibits tissue repair treatment effects.

Hereinafter, the present disclosure is set forth with specific preparation examples and examples. Abbreviations for compounds used in the description of preparation examples and examples are the following:

- mPEG: methoxy polyethylene glycol
- PCL: polycaprolactone

**Preparation Example 1: Preparation of mPEG2000-PCL2000 polymer formulation**

A copolymer (mPEG2000-PCL2000) was prepared by polymerizing methoxy polyethylene glycol, having a molecular weight of 2,000 g/mol, as a hydrophilic biocompatible polymer and polycaprolactone monomer, having a molecular weight of 2,000 g/mol, as a hydrophobic biocompatible polymer in the presence of a catalyst.

**Preparation Example 2: Preparation of mPEG2000-PCL4000 polymer formulation**

Preparation Example 2 was prepared using the same method as Preparation Example 1, except that polymerization is performed by using polycaprolactone having a molecular weight of 4,000 g/mol instead of polycaprolactone, having a molecular weight of 2,000 g/mol, in Preparation Example 1.

**Preparation Example 3: Preparation of mPEG2000-PCL5000 polymer formulation**

Preparation Example 3 was prepared using the same method as Preparation Example 1, except that polymerization is performed by using polycaprolactone having a molecular weight of 5,000 g/mol instead of polycaprolactone, having a molecular weight of 2,000 g/mol, in Preparation Example 1.

**Preparation Example 4: Preparation of mPEG2000-PCL7500 polymer formulation**

Preparation Example 4 was prepared using the same method as Preparation Example 1, except that polymerization is performed by using polycaprolactone having a molecular weight of 7,500 g/mol instead of polycaprolactone, having a molecular weight of 2,000 g/mol, in Preparation Example 1.

**Preparation Example 5: Preparation of mPEG2000-PCL10000 polymer formulation**

Preparation Example 5 was prepared using the same method as Preparation Example 1, except that polymerization is performed by using polycaprolactone having a molecular weight of 10,000 g/mol instead of polycaprolactone, having a molecular weight of 2,000 g/mol, in Preparation Example 1.

**Preparation Example 6: Preparation of mPEG2000-PCL12500 polymer formulation**

Preparation Example 6 was prepared using the same method as Preparation Example 1, except that polymerization is performed by using polycaprolactone having a molecular weight of 12,500 g/mol instead of polycaprolactone, having a molecular weight of 2,000 g/mol, in Preparation Example 1.

**Preparation Example 7: Preparation of mPEG2000-PCL15000 polymer formulation**

Preparation Example 7 was prepared using the same method as Preparation Example 1, except that polymerization is performed by using polycaprolactone having a molecular weight of 15,000 g/mol instead of polycaprolactone, having a molecular weight of 2,000 g/mol, in Preparation Example 1.

**Preparation Example 8: Preparation of mPEG5000-PCL5000 polymer formulation**

A copolymer (mPEG5000-PCL5000) was prepared by polymerizing methoxy polyethylene glycol, having a molecular weight of 5,000 g/mol, as a hydrophilic biocompatible polymer and polycaprolactone monomer, having a molecular weight of 5,000 g/mol, as a hydrophobic biocompatible polymer in the presence of a catalyst.

**Preparation Example 9: Preparation of mPEG5000-PCL7500 polymer formulation**

Preparation Example 9 was prepared using the same method as Preparation Example 8, except that polymerization is performed by using polycaprolactone having a molecular weight of 7,500 g/mol instead of polycaprolactone, having a molecular weight of 5,000 g/mol, in Preparation Example 8.

**Preparation Example 10: Preparation of mPEG5000-PCL10000 polymer formulation**

Preparation Example 10 was prepared using the same method as Preparation Example 8, except that polymerization is performed by using polycaprolactone having a molecular weight of 10,000 g/mol instead of polycaprolactone, having a molecular weight of 5,000 g/mol, in Preparation Example 8.

**Preparation Example 11: Preparation of mPEG5000-PCL12500 polymer formulation**

Preparation Example 11 was prepared using the same method as Preparation Example 8, except that polymerization is performed by using polycaprolactone having a molecular weight of 12,500 g/mol instead of polycaprolactone, having a molecular weight of 5,000 g/mol, in Preparation Example 8.

**Preparation Example 12: Preparation of mPEG5000-PCL15000 polymer formulation**

Preparation Example 12 was prepared using the same method as Preparation Example 8, except that polymerization is performed by using polycaprolactone having a molecular weight of 15,000 g/mol instead of polycaprolactone, having a molecular weight of 5,000 g/mol, in Preparation Example 8.

**Preparation Example 13: Preparation of mPEG5000-PCL17500 polymer formulation**

Preparation Example 13 was prepared using the same method as Preparation Example 8, except that polymerization is performed by using polycaprolactone having a molecular weight of 17,500 g/mol instead of polycaprolactone, having a molecular weight of 5,000 g/mol in Preparation Example 8.

**Preparation Example 14: Preparation of mPEG5000-PCL20000 polymer formulation**

Preparation Example 14 was prepared using the same method as Preparation Example 8, except that polymerization is performed by using polycaprolactone having a molecular weight of 20,000 g/mol instead of polycaprolactone, having a molecular weight of 5,000 g/mol in Preparation Example 8.

**Preparation Example 15: Preparation of mPEG5000-PCL25000 polymer formulation**

Preparation Example 15 was prepared using the same method as Preparation Example 8, except that polymerization is performed by using polycaprolactone having a molecular weight of 25,000 g/mol instead of polycaprolactone, having a molecular weight of 5,000 g/mol in Preparation Example 8.

**Preparation Example 16: Preparation of mPEG10000-PCL10000 polymer formulation**

A copolymer (mPEG10000-PCL10000) was prepared by polymerizing methoxy polyethylene glycol, having a molecular weight of 10,000 g/mol, as a hydrophilic biocompatible polymer and polycaprolactone monomer, having a molecular weight of 10,000 g/mol as a hydrophobic biocompatible polymer in the presence of a catalyst.

**Preparation Example 17: Preparation of mPEG10000-PCL12500 polymer formulation**

Preparation Example 17 was prepared using the same method as Preparation Example 16, except that polymerization is performed by using polycaprolactone having a molecular weight of 12,500 g/mol instead of polycaprolactone, having a molecular weight of 10,000 g/mol, in Preparation

Example 16.

**Preparation Example 18: Preparation of mPEG10000-PCL15000 polymer formulation**

Preparation Example 18 was prepared using the same method as Preparation Example 16, except that polymerization is performed by using polycaprolactone having a molecular weight of 15,000 g/mol instead of polycaprolactone, having a molecular weight of 10,000 g/mol, in Preparation Example 16.

**Preparation Example 19: Preparation of mPEG10000-PCL17500 polymer formulation**

Preparation Example 19 was prepared using the same method as Preparation Example 16, except that polymerization is performed by using polycaprolactone having a molecular weight of 17,500 g/mol instead of polycaprolactone, having a molecular weight of 10,000 g/mol, in Preparation Example 16.

**Preparation Example 20: Preparation of mPEG10000-PCL20000 polymer formulation**

Preparation Example 20 was prepared using the same method as Preparation Example 16, except that polymerization is performed by using polycaprolactone having a molecular weight of 20,000 g/mol instead of polycaprolactone, having a molecular weight of 10,000 g/mol, in Preparation Example 16.

**Preparation Example 21: Preparation of mPEG10000-PCL25000 polymer formulation**

Preparation Example 21 was prepared using the same method as Preparation Example 16, except that polymerization is performed by using polycaprolactone having a molecular weight of 25,000 g/mol instead of polycaprolactone, having a molecular weight of 10,000 g/mol, in Preparation Example 16.

**Preparation Example 22: Preparation of mPEG10000-PCL30000 polymer formulation**

Preparation Example 22 was prepared using the same method as Preparation Example 16, except that polymerization is performed by using polycaprolactone having a molecular weight of 30,000 g/mol instead of polycaprolactone, having a molecular weight of 10,000 g/mol, in Preparation Example 16.

**Example 1**

A colloidal aqueous solution having 5 wt% polymer was prepared by adding water to a polymer prepared by the Preparation Examples 1-22, heating to 80 °C, and mixing.

**Example 2**

A colloidal aqueous solution was prepared using the same method as Example 1, except that a colloidal aqueous solution having 10 wt% polymer was prepared.

**Example 3**

A colloidal aqueous solution was prepared using the same method as Example 1, except that a colloidal aqueous solution which has 15 wt% polymer was prepared.

**Example 4**

A colloidal aqueous solution was prepared using the same method as Example 1, except that a colloidal aqueous solution having 20 wt% polymer was prepared.

**Example 5**

A colloidal aqueous solution was prepared using the same method as Example 1, except that a colloidal aqueous solution having 25 wt% polymer was prepared.

**Example 6**

A colloidal aqueous solution was prepared using the same method as Example 1, except that a colloidal aqueous solution having 30 wt% polymer was prepared.

**Example 7**

A colloidal aqueous solution was prepared using the same method as Example 1, except that a colloidal aqueous solution having 35 wt% polymer was prepared.

**Example 8**

A colloidal aqueous solution was prepared using the same method as Example 1, except that a colloidal aqueous solution having 40 wt% polymer was prepared.

### Example 9

A colloidal aqueous solution was prepared using the same method as Example 1, except that a colloidal aqueous solution having 45 wt% polymer was prepared.

### Example 10

A colloidal aqueous solution was prepared using the same method as Example 1, except that a colloidal aqueous solution having 50 wt% polymer was prepared.

### Example 11

A colloidal aqueous solution was prepared using the same method as Example 1, except that a colloidal aqueous solution having 55 wt% polymer was prepared.

### Example 12

A colloidal aqueous solution was prepared using the same method as Example 1, except that a colloidal aqueous solution which has 60 wt% polymer was prepared.

### Example 13

A colloidal aqueous solution was prepared using the same method as Example 1, except that a colloidal aqueous solution having 65 wt% polymer was prepared.

### Experimental Example 1

The number of moles of a polymer in 100 g of an aqueous solution for the composition prepared according to the Examples 1-13 was measured, and the K factor according to the following equation 1 was measured. The effect of a formulation was evaluated according to this, the results are shown in Tables 1 and 2 below (the part of the effect of a formulation is highlighted).

<Equation 1>

$$K=(m_{100}*M_h^2*10)/(M_l*HLB^2)$$

In equation 1, $m_{100}$ is the number of moles of polymers in 100 g of the aqueous solution, $M_h$ is the molecular weight of a hydrophilic part, $M_l$ is the molecular weight of a hydrophobic part, and HLB is represented by the following equation 2,

<Equation 2>

$$HLB=20*M_h/M$$

In equation 2, $M_h$ is the molecular weight of the hydrophilic part, and M is the total molecular weight.

[Table 1]

| Concentration of Polymer in Aqueous solution (wt%) | | | 5% | 10% | 15% | 20% | 25% | 30% | 35% | 40% | 45% | 50% | 55% | 60% | 65% |
|---|---|---|---|---|---|---|---|---|---|---|---|---|---|---|---|
| | mPEG | PCL | HLB | | | | | | | | | | | | |
| Number of moles of Polymer in Aqueous solution 100 g | 2000 | 2000 | 10.00 | 0.0013 | 0.0025 | 0.0038 | 0.0050 | 0.0063 | 0.0075 | 0.0088 | 0.0100 | 0.0113 | 0.0125 | 0.0138 | 0.0150 | 0.0163 |
| | 2000 | 4000 | 6.67 | 0.0008 | 0.0017 | 0.0025 | 0.0033 | 0.0042 | 0.0050 | 0.0058 | 0.0067 | 0.0075 | 0.0083 | 0.0092 | 0.0100 | 0.0108 |
| | 2000 | 5000 | 5.71 | 0.0007 | 0.0014 | 0.0021 | 0.0029 | 0.0036 | 0.0043 | 0.0050 | 0.0057 | 0.0064 | 0.0071 | 0.0079 | 0.0086 | 0.0093 |
| | 2000 | 7500 | 4.21 | 0.0005 | 0.0011 | 0.0016 | 0.0021 | 0.0026 | 0.0032 | 0.0037 | 0.0042 | 0.0047 | 0.0053 | 0.0058 | 0.0063 | 0.0068 |
| | 2000 | 10000 | 3.33 | 0.0004 | 0.0008 | 0.0013 | 0.0017 | 0.0021 | 0.0025 | 0.0029 | 0.0033 | 0.0038 | 0.0042 | 0.0046 | 0.0050 | 0.0054 |
| | 2000 | 12500 | 2.76 | 0.0003 | 0.0007 | 0.0010 | 0.0014 | 0.0017 | 0.0021 | 0.0024 | 0.0028 | 0.0031 | 0.0034 | 0.0038 | 0.0041 | 0.0045 |
| | 2000 | 15000 | 2.35 | 0.0003 | 0.0006 | 0.0009 | 0.0012 | 0.0015 | 0.0018 | 0.0021 | 0.0024 | 0.0026 | 0.0029 | 0.0032 | 0.0035 | 0.0038 |
| | 5000 | 5000 | 10.00 | 0.0005 | 0.0010 | 0.0015 | 0.0020 | 0.0025 | 0.0030 | 0.0035 | 0.0040 | 0.0045 | 0.0050 | 0.0055 | 0.0060 | 0.0065 |
| | 5000 | 7500 | 8.00 | 0.0004 | 0.0008 | 0.0012 | 0.0016 | 0.0020 | 0.0024 | 0.0028 | 0.0032 | 0.0036 | 0.0040 | 0.0044 | 0.0048 | 0.0052 |
| | 5000 | 10000 | 6.67 | 0.0003 | 0.0007 | 0.0010 | 0.0013 | 0.0017 | 0.0020 | 0.0023 | 0.0027 | 0.0030 | 0.0033 | 0.0037 | 0.0040 | 0.0043 |
| | 5000 | 12500 | 5.71 | 0.0003 | 0.0006 | 0.0009 | 0.0011 | 0.0014 | 0.0017 | 0.0020 | 0.0023 | 0.0026 | 0.0029 | 0.0031 | 0.0034 | 0.0037 |
| | 5000 | 15000 | 5.00 | 0.0003 | 0.0005 | 0.0008 | 0.0010 | 0.0013 | 0.0015 | 0.0018 | 0.0020 | 0.0023 | 0.0025 | 0.0028 | 0.0030 | 0.0033 |
| | 5000 | 17500 | 4.44 | 0.0002 | 0.0004 | 0.0007 | 0.0009 | 0.0011 | 0.0013 | 0.0016 | 0.0018 | 0.0020 | 0.0022 | 0.0024 | 0.0027 | 0.0029 |
| | 5000 | 20000 | 4.00 | 0.0002 | 0.0004 | 0.0006 | 0.0008 | 0.0010 | 0.0012 | 0.0014 | 0.0016 | 0.0018 | 0.0020 | 0.0022 | 0.0024 | 0.0026 |
| | 5000 | 25000 | 3.33 | 0.0002 | 0.0003 | 0.0005 | 0.0007 | 0.0008 | 0.0010 | 0.0012 | 0.0013 | 0.0015 | 0.0017 | 0.0018 | 0.0020 | 0.0022 |
| | 10000 | 10000 | 10.00 | 0.0003 | 0.0005 | 0.0008 | 0.0010 | 0.0013 | 0.0015 | 0.0018 | 0.0020 | 0.0023 | 0.0025 | 0.0028 | 0.0030 | 0.0033 |
| | 10000 | 12500 | 8.89 | 0.0002 | 0.0004 | 0.0007 | 0.0009 | 0.0011 | 0.0013 | 0.0016 | 0.0018 | 0.0020 | 0.0022 | 0.0024 | 0.0027 | 0.0029 |
| | 10000 | 15000 | 8.00 | 0.0002 | 0.0004 | 0.0006 | 0.0008 | 0.0010 | 0.0012 | 0.0014 | 0.0016 | 0.0018 | 0.0020 | 0.0022 | 0.0024 | 0.0026 |
| | 10000 | 17500 | 7.27 | 0.0002 | 0.0004 | 0.0005 | 0.0007 | 0.0009 | 0.0011 | 0.0013 | 0.0015 | 0.0016 | 0.0018 | 0.0020 | 0.0022 | 0.0024 |
| | 10000 | 20000 | 6.67 | 0.0002 | 0.0003 | 0.0005 | 0.0007 | 0.0008 | 0.0010 | 0.0012 | 0.0013 | 0.0015 | 0.0017 | 0.0018 | 0.0020 | 0.0022 |
| | 10000 | 25000 | 5.71 | 0.0001 | 0.0003 | 0.0004 | 0.0006 | 0.0007 | 0.0009 | 0.0010 | 0.0011 | 0.0013 | 0.0014 | 0.0016 | 0.0017 | 0.0019 |
| | 10000 | 30000 | 5.00 | 0.0001 | 0.0003 | 0.0004 | 0.0005 | 0.0006 | 0.0008 | 0.0009 | 0.0010 | 0.0011 | 0.0013 | 0.0014 | 0.0015 | 0.0016 |

[Table 2]

| Concentration of Polymer in Aqueous solution (wt%) | | | 5% | 10% | 15% | 20% | 25% | 30% | 35% | 40% | 45% | 50% | 55% | 60% | 65% |
|---|---|---|---|---|---|---|---|---|---|---|---|---|---|---|---|
| | mPEG | PCL | HLB | | | | | | | | | | | | |
| K factor | 2000 | 2000 | 10.00 | 0.2500 | 0.5000 | 0.7500 | 1.0000 | 1.2500 | 1.5000 | 1.7500 | 2.0000 | 2.2500 | 2.5000 | 2.7500 | 3.0000 | 3.2500 |
| | 2000 | 4000 | 6.67 | 0.1875 | 0.3750 | 0.5625 | 0.7500 | 0.9375 | 1.1250 | 1.3125 | 1.5000 | 1.6875 | 1.8750 | 2.0625 | 2.2500 | 2.4375 |
| | 2000 | 5000 | 5.71 | 0.1750 | 0.3500 | 0.5250 | 0.7000 | 0.8750 | 1.0500 | 1.2250 | 1.4000 | 1.5750 | 1.7500 | 1.9250 | 2.1000 | 2.2750 |
| | 2000 | 7500 | 4.21 | 0.1583 | 0.3167 | 0.4750 | 0.6333 | 0.7917 | 0.9500 | 1.1083 | 1.2667 | 1.4250 | 1.5833 | 1.7417 | 1.9000 | 2.0583 |
| | 2000 | 10000 | 3.33 | 0.1500 | 0.3000 | 0.4500 | 0.6000 | 0.7500 | 0.9000 | 1.0500 | 1.2000 | 1.3500 | 1.5000 | 1.6500 | 1.8000 | 1.9500 |
| | 2000 | 12500 | 2.76 | 0.1450 | 0.2900 | 0.4350 | 0.5800 | 0.7250 | 0.8700 | 1.0150 | 1.1600 | 1.3050 | 1.4500 | 1.5950 | 1.7400 | 1.8850 |
| | 2000 | 15000 | 2.35 | 0.1417 | 0.2833 | 0.4250 | 0.5667 | 0.7083 | 0.8500 | 0.9917 | 1.1333 | 1.2750 | 1.4167 | 1.5583 | 1.7000 | 1.8417 |
| | 5000 | 5000 | 10.00 | 0.2500 | 0.5000 | 0.7500 | 1.0000 | 1.2500 | 1.5000 | 1.7500 | 2.0000 | 2.2500 | 2.5000 | 2.7500 | 3.0000 | 3.2500 |
| | 5000 | 7500 | 8.00 | 0.2083 | 0.4167 | 0.6250 | 0.8333 | 1.0417 | 1.2500 | 1.4583 | 1.6667 | 1.8750 | 2.0833 | 2.2917 | 2.5000 | 2.7083 |
| | 5000 | 10000 | 6.67 | 0.1875 | 0.3750 | 0.5625 | 0.7500 | 0.9375 | 1.1250 | 1.3125 | 1.5000 | 1.6875 | 1.8750 | 2.0625 | 2.2500 | 2.4375 |
| | 5000 | 12500 | 5.71 | 0.1750 | 0.3500 | 0.5250 | 0.7000 | 0.8750 | 1.0500 | 1.2250 | 1.4000 | 1.5750 | 1.7500 | 1.9250 | 2.1000 | 2.2750 |
| | 5000 | 15000 | 5.00 | 0.1667 | 0.3333 | 0.5000 | 0.6667 | 0.8333 | 1.0000 | 1.1667 | 1.3333 | 1.5000 | 1.6667 | 1.8333 | 2.0000 | 2.1667 |
| | 5000 | 17500 | 4.44 | 0.1607 | 0.3214 | 0.4821 | 0.6429 | 0.8036 | 0.9643 | 1.1250 | 1.2857 | 1.4464 | 1.6071 | 1.7679 | 1.9286 | 2.0893 |
| | 5000 | 20000 | 4.00 | 0.1563 | 0.3125 | 0.4688 | 0.6250 | 0.7813 | 0.9375 | 1.0938 | 1.2500 | 1.4063 | 1.5625 | 1.7188 | 1.8750 | 2.0313 |
| | 5000 | 25000 | 3.33 | 0.1500 | 0.3000 | 0.4500 | 0.6000 | 0.7500 | 0.9000 | 1.0500 | 1.2000 | 1.3500 | 1.5000 | 1.6500 | 1.8000 | 1.9500 |
| | 10000 | 10000 | 10.00 | 0.2500 | 0.5000 | 0.7500 | 1.0000 | 1.2500 | 1.5000 | 1.7500 | 2.0000 | 2.2500 | 2.5000 | 2.7500 | 3.0000 | 3.2500 |
| | 10000 | 12500 | 8.89 | 0.2250 | 0.4500 | 0.6750 | 0.9000 | 1.1250 | 1.3500 | 1.5750 | 1.8000 | 2.0250 | 2.2500 | 2.4750 | 2.7000 | 2.9250 |
| | 10000 | 15000 | 8.00 | 0.2083 | 0.4167 | 0.6250 | 0.8333 | 1.0417 | 1.2500 | 1.4583 | 1.6667 | 1.8750 | 2.0833 | 2.2917 | 2.5000 | 2.7083 |
| | 10000 | 17500 | 7.27 | 0.1964 | 0.3929 | 0.5893 | 0.7857 | 0.9821 | 1.1786 | 1.3750 | 1.5714 | 1.7679 | 1.9643 | 2.1607 | 2.3571 | 2.5536 |
| | 10000 | 20000 | 6.67 | 0.1875 | 0.3750 | 0.5625 | 0.7500 | 0.9375 | 1.1250 | 1.3125 | 1.5000 | 1.6875 | 1.8750 | 2.0625 | 2.2500 | 2.4375 |
| | 10000 | 25000 | 5.71 | 0.1750 | 0.3500 | 0.5250 | 0.7000 | 0.8750 | 1.0500 | 1.2250 | 1.4000 | 1.5750 | 1.7500 | 1.9250 | 2.1000 | 2.2750 |
| | 10000 | 30000 | 5.00 | 0.1667 | 0.3333 | 0.5000 | 0.6667 | 0.8333 | 1.0000 | 1.1667 | 1.3333 | 1.5000 | 1.6667 | 1.8333 | 2.0000 | 2.1667 |

Referring to Table 1, the number of moles of a polymer dissolved in 100 g of an aqueous solution can be seen, and it can be seen that the lower HLB value in a constant concentration, the fewer the number of moles.

When the concentration was more than 45 wt%, the viscosity of the colloidal aqueous solution was enhanced, so that it was hard to be injected through a syringe, and when the concentration was less than 15 wt%, the effect as a formulation was not exhibited.

Further, when HLB was less than 2.5, the rate of the hydrophobic biocompatible polymer was high, so that when water was added, the polymer was not dissolved, and when HLB was more than 10, the composition was absorbed in the body during the injection of the composition into the body, so that the effect as a formulation was not exhibited.

However, determining with not a proportion of a hydrophilic biopolymer and a hydrophobic biopolymer, but the molecular weight of a polymer polymerized in Table 1, comparing mPEG 2,000 g/mol and mPEG 5,000 g/mol, when mPEG was 2,000 g/mol, the number of moles of a polymer in 100 g of an aqueous solution increased more than when mPEG was 5,000 g/mol. Therefore, the number of moles of a polymer in 100 g of the aqueous solution was not constant, and thus converting this to a constant value, through this to measure a forming range of a composition for tissue repair treatment according to the present disclosure, resulting in K factor.

Referring to Table 2, unlike Table 1, a relationship of the molecular weight of a hydrophilic biopolymer and a hydrophobic biopolymer and HLB value may be understood, when the molecular weight of a hydrophilic biopolymer is the same, as the molecular weight of a hydrophobic biopolymer increases, K factor decreases. This is the same as determining with a proportion of a hydrophilic biopolymer and a hydrophobic biopolymer. Further, it can be seen that even though the molecular weight of a hydrophilic biopolymer is different, when a proportion of a hydrophilic biopolymer and a hydrophobic biopolymer is the same, the K factor has a very similar value.

The K factor has a constant value within a range of a certain concentration discussed below, the effect of a formulation within this range may be identified. In addition, the K factor is converted about 0.12-3.26, the part of the effect of a formulation is a range of 0.4-1.5.

Furthermore, it may be identified that the effect as a formulation is in a concentration of 15-45 wt% in a colloidal aqueous solution, HLB of 2.5-10, and K factor of 0.4-1.5.

**Experimental Example 2**

To measure a turbidity of a composition for tissue repair treatment according to the present disclosure as the following method, a colloidal phase prepared using Preparation Example 3, the result is shown in Table 3 below. Formazin turbidity standard, 4000NTU was used as a turbidity standard solution.

<Method of measuring>

(1) A comparable sample was prepared by making a solution in which a colloidal phase prepared using a standard solution and Preparation Example 3 was diluted 2-fold, 5-fold, 10-fold, and 20-fold, respectively, and putting this in a vial, and the turbidity of the standard solution diluted was 4,000, 2,000, 800, 400, and 200 NTU, respectively.

(2) After cleaning the outside of a vial of samples for comparing turbidity, changes in the turbidity due to dilution and difference of the turbidity in the same dilution rate was observed in a brightness of about 1000 lx below white LED light source.

[Table 3]

| | Undiluted (4000 NTU) | 2-fold dilution (2000 NTU) | 5-fold dilution (800 NTU) | 10-fold dilution (400 NTU) | 20-fold dilution (200 NTU) |
|---|---|---|---|---|---|
| Standard Solution | | | | | |
| Present Disclosure | | | | | |

EP 3 763 398 A1

Seeing the above Table 3, for the standard solution, from left to right, it can be seen that the more dilution the lower turbidity.

In contrast, for the present disclosure, from left to right, it can be identified with the naked eye that even though the solution was diluted, the turbidity did not decrease, and rather, the turbidity increased more than the undiluted solution.

**Experimental Example 3**

To validate the effect as a formulation of the composition for tissue repair treatment according to the present disclosure, animal experiments was performed.

Six week-old SD rats (purchased from Orient Bio) were used as an experimental animal.

The experiment was performed that the total 10 rats were subdivided into three groups by designating that one side is phosphate buffered saline (PBS) group, and the other is test sample group in eight sites per a six week-old SD rat individual. During experiment, feeding environment was set as a temperature of $24\pm2°C$, a relative humidity of $50\pm10\%$, and a lighting time of 12 hours, and animals was allowed to eat feeds freely.

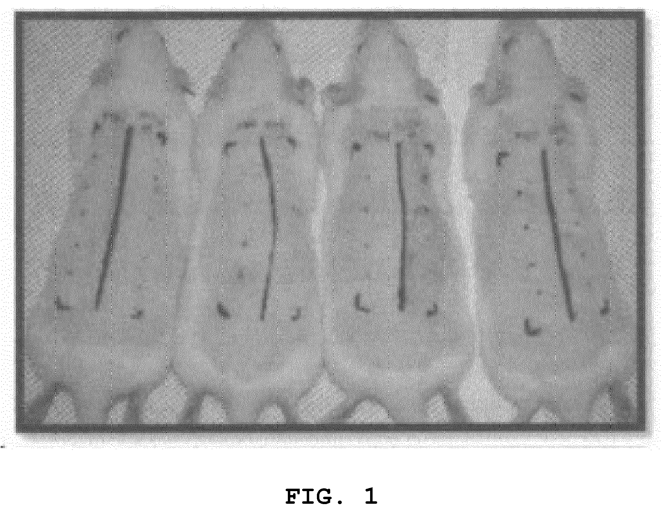

PBS was injected to the left subcutaneous layer with respect to the center line of a rat in each group, and a colloidal aqueous solution prepared by dissolving a polymer in water, which was prepared in the Preparation Example 3 (which has a concentration of 25%, HLB of 5.7, K factor of 0.8864) is regularly injected at 250 $\mu\ell$. Immediately after injection, it was observed whether the samples were leaked, and the results are shown in Fig. 1.

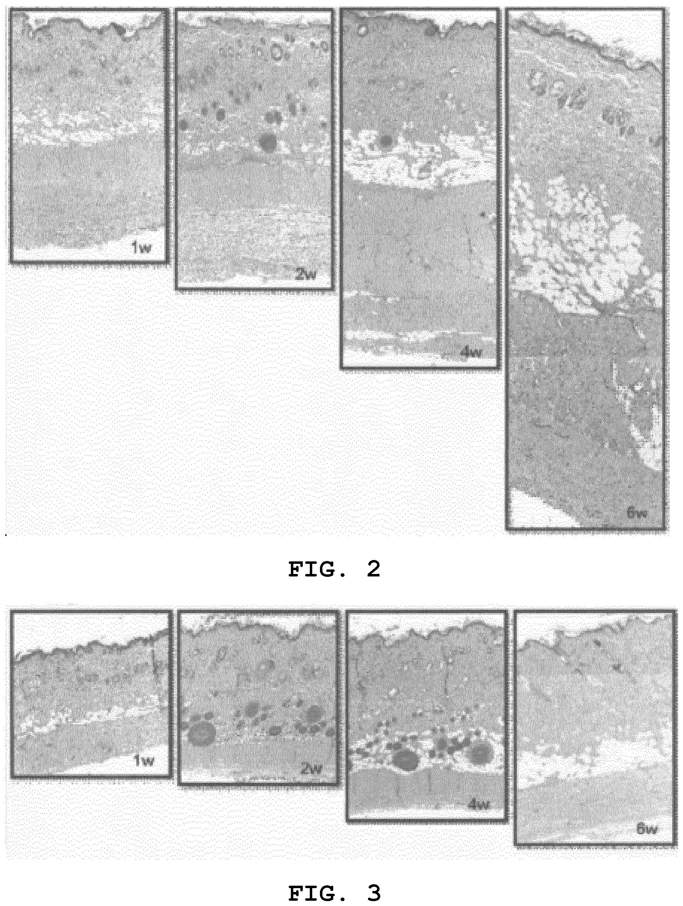

Immediately after administering the colloidal aqueous solution and PBS (0 hr), the experimental animals were sacrificed after one week, two weeks, four weeks, and six weeks, respectively, the skin tissues in which the samples were injected and the skin tissues in which the samples were not injected were harvested and fixed in 10% neutral buffered formalin solution. Then, the skin tissues were embedded in paraffin and solidified, and 5 $\mu$m sections were prepared. The sections were stained with Hematoxylin and Eosin (H&E), and inflammation/foreign substance reaction was then evaluated according to Table 4 below. Thickness increase of the whole skin layer (dermal layer and subcutaneous layer) due to the sample injection was observed through an optical microscope, and the results are shown in Figs. 2, 4, and 6, respectively.

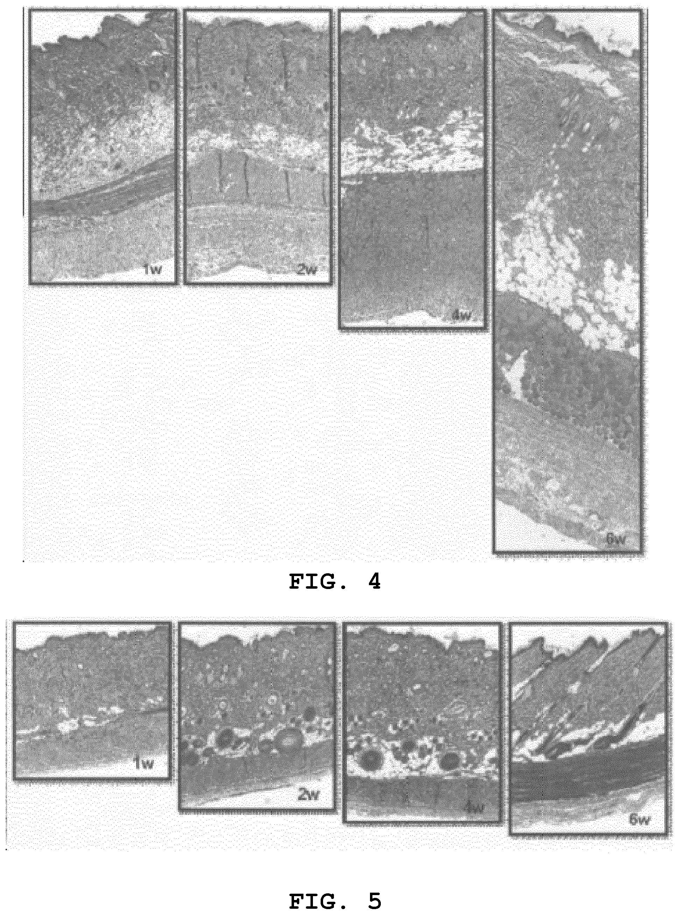

In addition, to evaluate a biosynthesis ability of new collagen of a colloidal aqueous solution and PBS, the sections were stained with Masson's Trichorme (MT), and collagen formation in the tissue was then observed. Histocompatibility of the samples injected was evaluated through confirming inflammation and foreign substance reaction according to Table 4 below as major criteria. Tissue slides were observed with 40x, 100x, 200x, and 400x using an optical microscope, major histological features of each slide were deciphered, and the results are shown in Figs. 3 and 5.

Further, the degree of inflammation and foreign substance reaction due to the colloidal aqueous solution injected is divided into four stages. Inflammation and foreign substance reaction which is observed in PBS-administered group is set as no inflammation, and as inflammation reaction or foreign substance reaction is intensified, the degree is set as almost clear (score 1), mild(score 2), moderate(score 3), severe(score 4) and evaluated according to Table 4 below (Duranti et al. Dermatol Surg 1998:24:1317-25).

[Table 4]

|  | Grade | Foreign body granuloma |
|---|---|---|
| score 0 | No inflammation | No visible reaction |
| score 1 | almost clear | Slight reaction with a few inflammatory cells |
| score 2 | mild | Clear inflammatory reaction with one or two giant cells |
| score 3 | moderate | Fibrous tissue with inflammatory cells, lymphocytes and giant cells |
| score 4 | severe | Granuloma with encapsulated implant-clear foreign body reaction |

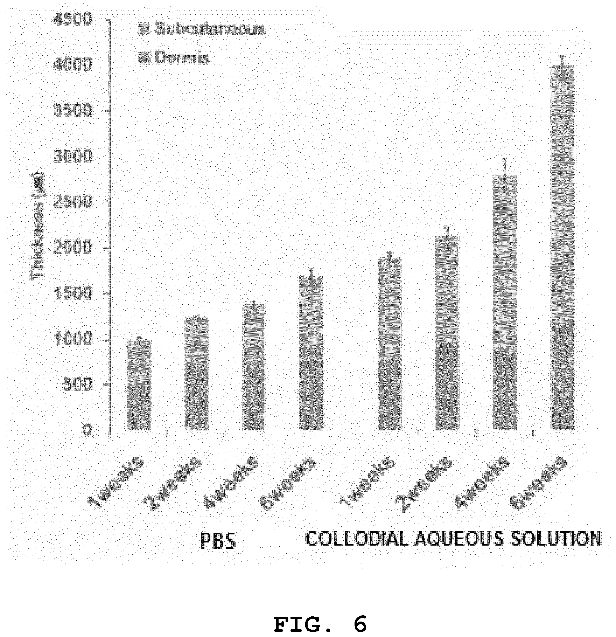

Fig. 1 is a picture which was taken with DSLR (D3000, Nikon, Japan) to confirm whether samples were leaked after PBS and a colloidal aqueous solution was injected; Fig. 2 is a picture, which was taken through an optical microscope, of skin thickness over time after a colloidal aqueous solution was injected; and Fig. 3 is a picture, which was taken through an optical microscope, of collagen over time after a colloidal aqueous solution was injected. Fig. 4 is a picture, which was taken through an optical microscope, of skin thickness over time after PBS was injected; Fig. 5 is a picture, which was taken through an optical microscope, of skin thickness over time after PBS was injected; and Fig. 6 is a graph showing skin thickness over time after PBS and a colloidal aqueous solution was injected.

Referring to Fig. 1, when being observed immediately after injecting PBS and the colloidal aqueous solution, it may be identified that the samples were not leaked.

Referring to Figs. 2, 4, and 6, as a result of histopathological evaluation with H&E stain, it may be identified that the thickness of subcutaneous layer in the tissue subcutaneous layer in which the colloidal aqueous solution was injected also increased as the colloidal aqueous solution was injected over time up to six weeks, and it can be seen that an amount of increase according to this was certainly improved relative to Fig. 4 in which PBS was injected.

Referring to Figs. 3 and 5, as a result of histopathological evaluation with MT stain, it may be identified that the collagen formation in the tissue subcutaneous layer in which the colloidal aqueous solution was injected was identified, and the thickness of subcutaneous layer according to the collagen formation also increased over time up to six weeks, and it can be seen that an amount of increase according to this was certainly improved relative to Fig. 5 in which PBS was injected.

Further, referring to Figs. 2-5, when evaluating foreign substance reaction according to Table 4 above, it was identified that a significant foreign substance reaction due to the colloidal aqueous solution injection was not observed, and inflammatory cells, lymphocytes, and macrophages in a fibrous tissue were hardly seen (Score 1), there was no difference in foreign substance reaction before the colloidal aqueous solution injection.

As such, when meeting a concentration, HLB, and K value according to the present disclosure, a composition for tissue repair treatment using a non-toxic biocompatible polymer and a method for manufacturing the same may be provided.

Hitherto, the preferred examples of the present disclosure have been described with reference to figures. Although the examples of the present disclosure have been disclosed for illustrative purposes, those skilled in the art will appreciate that various modifications, additions, and substitutions are possible, without departing from the technical idea or essential features of the present disclosure.

Accordingly, the scope of the present disclosure is defined by the following claims rather than by the detailed description of the examples. It shall be understood that all modifications or changes in forms conceived from the meaning and scope of the claims and their equivalents are included in the scope of the present disclosure.

**Claims**

1.   A composition for tissue repair treatment, comprising a copolymer in which a hydrophobic biocompatible polymer and a hydrophilic biocompatible polymer are polymerized, the composition having a colloidal phase in which the copolymer is dispersed in water.

2.   The composition for tissue repair treatment of claim 1, wherein the composition has a range of K factor represented by the following equation 1 is 0.3-1.8:

$$\text{<Equation 1>}$$

$$K = (m_{100} * M_h^2 * 10)/(M_l * HLB^2)$$

where $m_{100}$ is the number of moles of polymers in 100 g of an aqueous solution, $M_h$ is the molecular weight of a hydrophilic part, $M_l$ is the molecular weight of a hydrophobic part, and HLB is represented by the following equation 2,

$$\text{<Equation 2>}$$

$$HLB = 20 * M_h / M$$

where $M_h$ is the molecular weight of the hydrophilic part, and M is the total molecular weight.

3.   The composition for tissue repair treatment of claim 1, wherein the value of HLB is 1-14 in the equation 2.

4.   The composition for tissue repair treatment of claim 1, wherein the hydrophobic biocompatible polymer is at least any one polymer selected from the group consisting of polyglycolic acid, polycaprolactone, poly lactic acid, polydioxanone, poly(trimethylene carbonate), polyhydroxybutyrate, and a copolymer including the same.

5.   The composition for tissue repair treatment of claim 1, wherein the hydrophilic biocompatible polymer comprises a glycol compound.

6. The composition for tissue repair treatment of claim 5, wherein the glycol compound is at least any one polymer selected from the group consisting of methoxy polyethylene glycol, dihydroxy polyethylene glycol, mono-alkoxy polyethylene glycol, and polyethylene glycol.

7. The composition for tissue repair treatment of claim 1, wherein the bonding structure of the copolymer comprises the structure of the following formula 1, formula 2, or formula 3:

[Formula 1]

X-Y

[Formula 2]

Y-X-Y

[Formula 3]

X-Y-X

where X is the hydrophilic biocompatible polymer, and Y is the hydrophobic biocompatible polymer.

8. The composition for tissue repair treatment of claim 1, wherein the hydrophilic biocompatible polymer is 300-20,000 g/mol.

9. The composition for tissue repair treatment of claim 1, wherein the hydrophobic biocompatible polymer is 1,000-30,000 g/mol.

10. The composition for tissue repair treatment of claim 1, wherein the copolymer is 1,300-50,000 g/mol.

11. The composition for tissue repair treatment of claim 1, wherein the concentration of the copolymer in a colloidal solution is 10-50 wt%.

12. The composition for tissue repair treatment of claim 1, wherein the colloidal solution has no change or an increase in turbidity when water is added.

13. A method for manufacturing a composition for tissue repair treatment, the method comprising:

   preparing a polymer by polymerizing a hydrophobic biocompatible polymer and a hydrophilic biocompatible polymer; and
   obtaining a colloidal solution by adding the polymer to water.

FIG. 1

FIG. 2

FIG. 3

FIG. 4

FIG. 5

FIG. 6

FIG. 7

| INTERNATIONAL SEARCH REPORT | International application No. |
|---|---|
| | **PCT/KR2018/005988** |

**A. CLASSIFICATION OF SUBJECT MATTER**

*A61L 27/60(2006.01)i, A61L 27/40(2006.01)i, A61L 27/14(2006.01)i, C08L 101/00(2006.01)i, C08J 3/05(2006.01)i, C08G 81/00(2006.01)i*

According to International Patent Classification (IPC) or to both national classification and IPC

**B. FIELDS SEARCHED**

Minimum documentation searched (classification system followed by classification symbols)

A61L 27/60; A61F 2/10; A61K 47/30; A61K 9/08; A61L 27/14; C08L 101/16; A61L 27/40; C08L 101/00; C08J 3/05; C08G 81/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: polymerization, hydrophilicity, hydrophobicity, tissue repair, colloid

**C. DOCUMENTS CONSIDERED TO BE RELEVANT**

| Category* | Citation of document, with indication, where appropriate, of the relevant passages | Relevant to claim No. |
|---|---|---|
| X | KR 10-2016-0107780 A (DEXLEVO INC. et al.) 19 September 2016<br>See claims 1-9; paragraphs [0016] to [0051], [0109]. | 1-12 |
| X | KR 10-2016-0122111 A (DEXLEVO INC.) 21 October 2016<br>See the entire document. | 1,4-12 |
| A | LEE, Young Moo et al. Methoxy poly(ethylene glycol) and ε -caprolactone amphiphilic block copolymeric micelle containing indomethacin. II. Micelle formation and drug release behaviours. Journal of Controlled Release. 1998, vol. 51, no. 1, pages 13-22<br>See the entire document. | 1-12 |
| A | KR 10-2015-0007051 A (YU, Jae Won) 20 January 2015<br>See the entire document. | 1-12 |
| A | KR 10-2015-0010464 A (YU, Jae Won) 28 January 2015<br>See the entire document. | 1-12 |

☐ Further documents are listed in the continuation of Box C.     ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 04 APRIL 2019 (04.04.2019) | **05 APRIL 2019 (05.04.2019)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

<table>
<tr><td colspan="2"><b>INTERNATIONAL SEARCH REPORT</b><br>Information on patent family members</td><td colspan="2">International application No.<br><b>PCT/KR2018/005988</b></td></tr>
</table>

| Patent document cited in search report | Publication date | Patent family member | Publication date |
|---|---|---|---|
| KR 10-2016-0107780 A | 19/09/2016 | KR 10-1689357 B1 | 26/12/2016 |
| KR 10-2016-0122111 A | 21/10/2016 | KR 10-1689798 B1<br>KR 10-2016-0033897 A<br>WO 2016-043547 A1 | 27/12/2016<br>29/03/2016<br>24/03/2016 |
| KR 10-2015-0007051 A | 20/01/2015 | KR 10-2015-0048683 A | 07/05/2015 |
| KR 10-2015-0010464 A | 28/01/2015 | KR 10-1531091 B1<br>WO 2015-009035 A1 | 24/06/2015<br>22/01/2015 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020140072008

**Non-patent literature cited in the description**

- **KLAUS LAESCHKE.** Biocompatibility of Microparticles into Soft Tissue Fillers. *Semin Cutan Med Surg 23*, 2004, 214-217

- **DURANTI et al.** *Dermatol Surg,* 1998, vol. 24, 1317-25